Europäisches Patentamt

European Patent Office

Office européen des brevets

(11) Veröffentlichungsnummer: **0 311 094 B1**

(12) **EUROPÄISCHE PATENTSCHRIFT**

(45) Veröffentlichungstag der Patentschrift: **27.04.94**

(21) Anmeldenummer: **88116637.5**

(22) Anmeldetag: **07.10.88**

(51) Int. Cl.⁵: **G01N 33/92**, G01N 33/546

(54) Diagnostisches Mittel und Verfahren zur Bestimmung von Apolipoprotein B.

(30) Priorität: **08.10.87 DE 3734015**

(43) Veröffentlichungstag der Anmeldung:
**12.04.89 Patentblatt 89/15**

(45) Bekanntmachung des Hinweises auf die
Patenterteilung:
**27.04.94 Patentblatt 94/17**

(84) Benannte Vertragsstaaten:
**AT BE CH DE ES FR GB IT LI LU NL SE**

(56) Entgegenhaltungen:
**EP-A- 0 001 223**
**EP-A- 0 262 854**
**WO-A-86/05493**

**CHEMICAL ABSTRACTS, Band 102, Nr. 21,
Mai 1985, Columbus, OH (US); N.V. DAC et
al., Seiten 289-290, Nr. 181710z&NUM;**

**CHEMICAL ABSTRACTS, Band 104, Nr. 9,
März 1986, Columbus, OH (US); Seite 400, Nr.
75106p&NUM;**

(73) Patentinhaber: **BEHRINGWERKE Aktiengesellschaft**
**Postfach 1140**
**D-35001 Marburg(DE)**

(72) Erfinder: **Schmidtberger, Rudolf**
**Salegrund 1**
**D-3550 Marburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

EP 0 311 094 B1

**Beschreibung**

Es wird ein diagnostisches Mittel enthaltend eine Dispersion von Partikeln, an die Antikörper gegen Apolipoprotein B gebunden sind, und ein Verfahren unter Verwendung dieses Mittels zur Bestimmung von Apolipoprotein B beschrieben.

Der Transport der an sich wasserunlöslichen Lipide im Stoffwechsel des Menschen geschieht in Form der sogenannten Lipoproteine. Von diesen, aus Proteinen und Lipiden bestehenden Komplexen ist in den letzten Jahren die Klasse mit geringer Dichte, die sogenannten Low Density Lipoproteine (LDL) als Indikator für eine coronare Erkrankung erkannt worden.

Die Tatsache, daß die Klasse der LDL im Gegensatz zu den übrigen Lipoproteinen als Proteinkomponente nur Apolipoprotein B enthält, ist für eine immunchemische Bestimmung in Form einer (nephelometrischen) Trübungsreaktion und in einer Kombination von immunadsorptiver Trennung der LDL von den übrigen Lipoproteinen und anschließender Bestimmung eines geeigneten Bestandteils der Lipide genutzt worden (DE 32 15 310). Verfahren zur immunchemischen Bestimmung von Apolipoproteinen A (HDL) werden in der WO 86/05493 und in CHEMICAL ABSTRACTS 104(9) Nr. 75106p beschrieben. Für diese Verfahren ist jedoch eine entsprechende apparative Ausstattung nötig.

Es bestand daher das Bedürfnis für ein einfaches Verfahren für die Bestimmung von Apolipoprotein B.

Es wurde überraschenderweise gefunden, daß ein Agglutinationsreagenz für die immunologische Bestimmung von Apolipoprotein B hergestellt werden kann, das eine schnelle Bestimmung dieses Proteins ohne apparativen Aufwand erlaubt, und das vorteilhafterweise mit lipämischen Proben sowie Vollblut, das nicht zu Plasma oder Serum aufgearbeitet werden muß, verwendet werden kann.

Die Erfindung betrifft ein Mittel zur Bestimmung von Apolipoprotein B (Apo B) in Blut, Plasma oder Serum mittels eines Agglutinationsverfahrens, enthaltend eine Dispersion von Partikeln, an die Antikörper gegen Apo B gebunden sind.

An die Partikel kann ein Antikörper gegen Apo B durch adsorptive oder kovalente oder auch über eine bioaffine Bindung, beispielsweise durch Staphylococcus aureus Protein A, welches zuerst auf die Partikel gebracht wird, gebunden werden. Die mit dem Antikörper beladenen Partikel werden als Dispersion hergestellt und gegebenenfalls mit Zusätzen wie Proteinen oder Detergentien versetzt, um spontane Agglutination zu vermeiden.

Ein solches Mittel kann Partikel mineralischer Natur wie Bentonit, Kunststoffkügelchen wie Latices mit einem Durchmesser von 40 bis 1200 nm, aus quervernetzten Polysacchariden oder auch aus Erythrozyten, deren Oberfläche durch chemische Behandlung gegen unterschiedliche osmotische Verhältnisse der sie umgebenden Lösungen unempfindlich gemacht wurde, in einem wässrigen Medium enthalten.

Die Menge an Antikörper wird so gewählt, daß bei einer Konzentration von Apo B, die klinisch als hoch zu bezeichnen ist, die Agglutination zu einem späteren Zeitpunkt eintritt als bei niederer Konzentration.

Die Erfindung betrifft weiterhin ein Verfahren gemäß Anspruch 5 zur Bestimmung der Konzentration von Apo B in einer Probe von Blut, Plasma oder Serum, indem man die Probe mit einer Dispersion von Partikeln, die an die Antikörper gegen Apolipoprotein B gebunden sind, mischt, indem man die Zeit bis zur Agglutination bestimmt und die Konzentration von Apo B einer Vergleichskurve entnimmt.

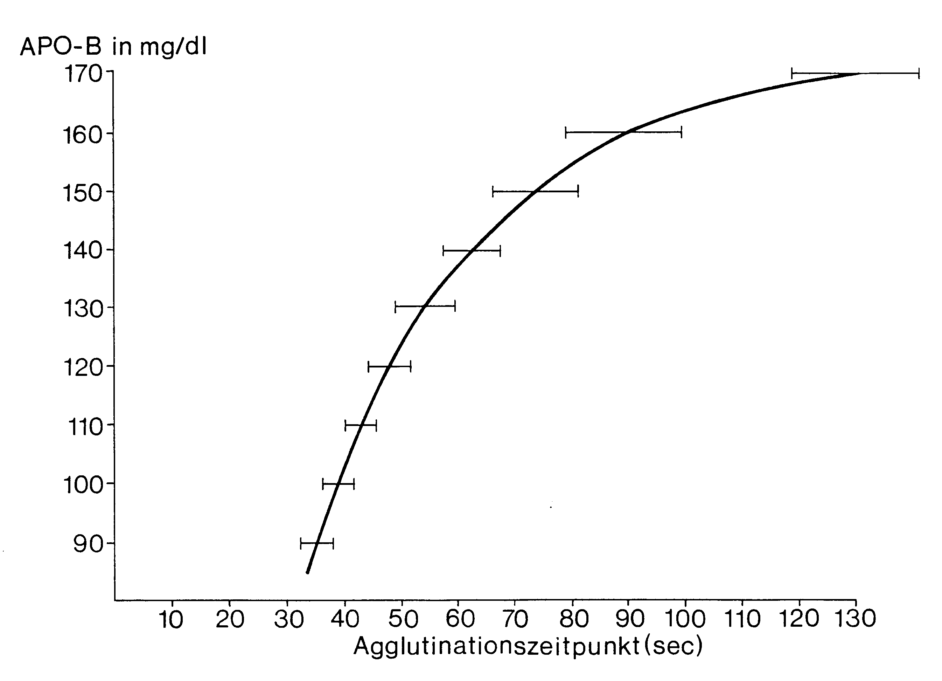

Figur 1 zeigt eine typische Vergleichskurve. Auf der senkrechten Achse ist die Menge an Apo B und auf der waagrechten der gemessene Agglutinationszeitpunkt aufgetragen. Die auf der Kurve eingezeichneten Strecken stellen die Variationsbreite der gemessenen Zeitpunkte dar.

Vorzugsweise wird die Beladung der Partikel und das Mischungsverhältnis von Dispersion und Probe so gewählt, daß die Mischung nach zwei Minuten agglutiniert, wenn die Probe 170 mg Apo B in 100 ml enthält. Wenn die Partikel nach zwei Minuten (oder früher) agglutiniert sind, bedeutet dies dann, daß die Konzentration an Apo B kleiner als 170 mg/100 ml und damit im klinischen Normalbereich ist, während eine nach Ablauf von zwei Minuten fehlende Agglutination eine erhöhte, also über dem klinischen Normalbereich liegende Apo B-Konzentration anzeigt.

Ein geeigneter Antikörper kann durch Immunisierung einer geeigneten Tierspezies mit humanem Apo B erzeugt und aus dem Serum des oder der jeweiligen Tiere nach bekannten Methoden beispielsweise durch Ausfällung, durch Ionenaustauschchromatographie oder Gelfiltration oder durch Kombination solcher Verfahren gewonnen werden. Werden monoklonale Antikörper verwendet, sind mindestens zwei Klone zu selektieren, die Antikörper gegen zwei räumlich voneinander getrennte Epitope des Apo B sezernieren, wobei diese monoklonalen Antikörper in einem geeigneten, zum Beispiel einem experimentell zu ermittelnden Mengenverhältnis an die Partikel gebunden sind.

Die Menge des oder der Antikörper mit einem bestimmten Titer, die an eine definierte Menge Partikel gebunden ist, ist ausschlaggebend für den Zeitpunkt der Agglutination. Sie wird so gewählt, daß bei einer beispielsweise durch einen Standard vorgegebenen Konzentration von Apo B die Agglutination zu einem

2

früheren Zeitpunkt stattfindet, wenn wenig Antikörper und zu einem späteren Zeitpunkt, wenn mehr Antikörper an die Partikel gebunden sind.

Bevorzugte Ausführungsformen des diagnostischen Mittels enthalten 5 bis 15 vorzugsweise 8 bis 10 mg/ml dispergiertes Polystyrol-Latex mit Partikelgrößen von 200 bis 800 vorzugsweise 500 bis 700 nm und Antikörper in einer Konzentration von 2,2 bis 3,3 vorzugsweise 2,5 bis 2,7 mg/ml mit Titereinheiten von 4 bis 6 vorzugsweise 5. Die Definition einer Titereinheit als 1 mg Apo B/ml Antiserum ist für die radiale Immunodiffusion beschrieben in Immunochemistry 6, 539-546 (1969), insbesondere auf Seite 540 in Gleichung (3).

Eine besonders bevorzugte Ausführungsform des diagnostischen Mittels enthält 9 mg/ml Polystyrol-Latex mit einer Partikelgröße von 600 nm sowie IgG Antikörper vom Kaninchen in einer Konzentration von 2,6 mg/ml mit einem Titer von 5 Einheiten/ml.

Die Beladung der Partikel mit Antikörper wird zweckmäßigerweise so gewählt, daß bei einer Konzentration von 150 mg/100 ml Apo B die Agglutination nach 75 Sekunden eintritt. Um das diagnostische Mittel derart einzustellen, werden an Aliquote von Partikeln unterschiedlichen Mengen Antikörper gebunden und die daraus hergestellten Mittel mit einem Standard von 150 mg/100 ml Apo B reagieren lassen. Die Mittel, welche mit dem Standard nach 70 bis 80 Sekunden agglutinieren, geben die Orientierung für die Menge an Antikörper, die zur Bindung an die Partikel erforderlich ist, um eine Agglutination nach 75 Sekunden zu erreichen. Der beschriebene orientierende Versuch für die Herstellung des Mittels ist mit jeder anderen Antikörperpräparation zu wiederholen, wenn diese sich nach Gewinnung aus der gleichen Tierspezies im Titer, oder bei gleichem Titer und Gewinnung in einer anderen Tierspezies, oder durch beide Gegebenheiten unterscheidet.

Das in der beschriebenen Weise hergestellte diagnostische Mittel wird zusätzlich mit weiteren Standards von Apo B, daß heißt mit solchen niederer und höherer Konzentrationen als 150 mg/100 ml getestet und daraus die zeitliche Abhängigkeit der Agglutination von der Konzentration ermittelt. Eine bei einem solchen Verfahren erhaltene Standardkurve ist beispielhaft in der Figur dargestellt. Mit Hilfe einer solchen Vergleichskurve ist es möglich, Apo B innerhalb des durch die Kurve vorgegebenen Konzentrationsbereichs quantitativ zu bestimmen.

Die Erfindung wird durch folgendes Beispiel näher erläutert, ohne sie jedoch darauf zu beschränken.

Beispiel

1. Diagnostisches Mittel
1.1 Antikörper gegen Apo B
Aus menschlichem Serum wurden durch Ultrazentrifugation die Lipoproteine der Dichteklasse 1.019 - 1.063 gewonnen (Havel, R.J., Eder, H.A. and Bragdon, J.H. (1955) J.Clin.Invest. 34, 1345).
Mit diesen so gewonnenen LDL wurden in Kaninchen Antikörper gegen die Proteinkomponente, das Apolipoprotein B erzeugt. Die Immunisierung der Tiere wurde solange fortgeführt, bis bei Probentnahmen ein durchschnittlicher Titer von 19 Einheiten/ml im Serum dieser Tiere gemessen wurde.
Ein Pool aus den Seren mehrerer Tiere wurde gegen 30 mmol/l Natrium-Phosphat-Puffer pH 7.0 dialysiert. Anschließend wurde durch Chromatographie an DEAE-Cellulose DE 32 (Whatman), die mit dem gleichen Puffer äquilibriert worden war, die Gammaglobulin-Fraktion gewonnen. Dazu wurde der die Gammaglobuline enthaltende Säulendurchfluß bis zur 50 % Sättigung mit festem Ammoniumsulfat versetzt. Die im Präzipitat enthaltenen Gammaglobuline wurden durch Gelfiltration von Ammoniumsulfat befreit und dabei gleichzeitig in eine Lösung überführt, die 50 mmol/l NaCl, 100 mmol/l Glycin und 15 mmol/l Natriumazid enthielt und deren pH-Wert auf 8,2 mit NaOH eingestellt worden war. Die Proteinkonzentration der Lösung wurde anschließend durch Ultrafiltrieren auf 50 mg/ml gebracht. Parallel dazu wurde eine Humanalbumin-Lösung durch Gelfiltration in die gleiche NaCl-Glycin-Lösung überführt. Sie hatte danach einen Proteingehalt von 120 mg/ml.
1.2 Latex-Agglutinationsreagenz
2.0 ml Gammaglobulin-Lösung und 4.1 ml Albumin-Lösung wurden gemischt und mit NaCl-Glycin-Lösung auf 20.4 ml aufgefüllt. Die Lösung wurde anschließend 30 min auf 56 °C erwärmt.
Die so vorbehandelte Lösung wurde dann unter Rühren mit einer Dispersion von 20 mg/ml Polystyrol-Latex Partikel von 600 nm Durchmesser in NaCl-Glycin-Lösung versetzt. Insgesamt wurden 17.5 ml der Latex-Dispersion mit einer Geschwindigkeit von 1 ml/h zugegeben.
2. Verfahren zur Bestimmung von Apolipoprotein B
2.1 Erstellung einer Standardkurve
Durch spontane Gerinnung frisch gewonnnenes Humanserum wurde 30 min bei 500 000xg zentrifugiert. Die dabei flotierten Chylomikronen wurden abgesaugt und verworfen. Das so geklärte Serum wurde mit Natriumchlorid bis zu einer Dichte von 1.063 versetzt und anschließend 30 min bei 500 000xg zentrifugiert. Der Inhalt des Zentrifugenbechers wurde in eine obere, LDL-reiche und eine untere, LDL-arme Fraktion geteilt. In beiden Fraktionen wurde immunchemisch der Apo B-Gehalt bestimmt:

| obere Fraktion: | 234 mg/100 ml |
|---|---|
| untere Fraktion : | 38 mg/100 ml |

Durch Mischen bestimmter rechnerisch ermittelter Volumina beider Lösungen wurden die zur Erstellung der Eichkurve notwendigen Lösungen mit abgestuftem Apo B-Gehalt erhalten.

20 $\mu$l eines Humanserums mit 150 mg/100 ml Apo B ergaben mit 20 $\mu$l Latex-Reagenz zur Reaktion gebracht nach 75 sec eine Agglutination.

2.2 Vergleichende Bestimmung von Apo B in Vollblut, Plasma und Serum desselben Spenders

Blut, von einem gesunden männlichen Spender, wurde in einer Natriumcitrat enthaltenden Vorlage gesammelt. Ein Drittel des Blutes wurde als Vollblut belassen. Die restlichen zwei Drittel wurden zur Abtrennung der geformten Blutbestandteile zentrifugiert. Das als Überstand vorliegende Plasma wurde gewonnen und halbiert. Die eine Plasmahälfte wurde mit 2 IU Thrombin/ml Plasma versetzt und nach Beendigung des Gerinnungsvorganges durch zentrifugieren von dem ausgefallenen Fibrin befreit. Das als Zentrifugenüberstand vorliegende Serum wurde dekantiert und anschließend vergleichend mit Vollblut und Plasma untersucht. Unter Berücksichtigung des vorher für das Vollblut bestimmten Hämatokrits von 43 wurden 35 $\mu$l Vollblut, 20 $\mu$l Plasma und 20 $\mu$l Serum mit je 20 $\mu$l Latexreagenz zur Reaktion gebracht. Die gefundenen Agglutinationszeiten waren

```
Vollblut   63 sec entsprechend 140 mg Apo B/100 ml
Plasma     59  "         "        136  "   "     "
Serum      55  "         "        132  "   "     "
```

Vergleichend dazu wurde der Apo B-Gehalt mit dem Behring-Nephelometer Analyser bestimmt. Bei dieser nephelometrischen Bestimmung wurden für das Serum 137 mg Apo B/100 ml gefunden.

2.3 Vergleichende Bestimmung von Apo B in lipämischen und nicht lipämischen Serumproben desselben Spenders Serum, gewonnen aus lipämischem Plasma durch Auslösen der Gerinnung mit 2 IU/ml Thrombin, wurde 30 Minuten bei 504 000xg (Beckman-Zentrifuge, Rotor 70 TI) zentrifugiert. Nach Entfernen der Chylomikronen durch Abhebern der oberen getrübten Zone wurde der Inhalt des Zentrifugenröhrchens wieder gemischt und vergleichend mit einer Probe des unzentrifugierten lipämischen Serums dem Latex-Test unterzogen. Beide Proben agglutinierten zum gleichen Zeitpunkt, nämlich nach 95 sec, entsprechend 162 mg Apo B/100 ml, wie aus der Standardkurve abgelesen wurde.

**Patentansprüche**
**Patentansprüche für folgende Vertragsstaaten : AT, BE, CH, DE, FR, GB, IT, LI, LU, NL, SE**

1. Mittel zur Bestimmung von Apolipoprotein B (Apo B) in Blut, Plasma oder Serum mittels eines Agglutinationsverfahrens, enthaltend eine Dispersion von Partikeln, an die Antikörper gegen Apo B gebunden sind und gegebenenfalls stabilisierende Zusätze dadurch gekennzeichnet, daß die Menge an Antikörper so gewählt wird, daß bei einer Konzentration von Apo B, die oberhalb des klinischen Normalbereichs liegt, die Agglutination zu einem späteren Zeitpunkt eintritt als die Agglutination bei einer Konzentration im klinischen Normalbereich.

2. Mittel nach Anspruch 1, dadurch gekennzeichnet, daß es zusätzlich ein Protein oder ein Detergens enthält.

3. Mittel nach Anspruch 1, dadurch gekennzeichnet, daß es 5 bis 15 mg/ml dispergiertes Polystyrol-Latex mit einer Partikelgröße von 200 bis 800 nm und Antikörper in einer Konzentration von 2,2 bis 3,3 mg/ml mit einer Titereinheit von 4 bis 6 enthält.

4. Mittel nach Anspruch 1, dadurch gekennzeichnet, daß es 8 bis 10 mg/ml Polystyrol-Latex mit einer Partikelgröße von 500 bis 700 nm sowie IgG Antikörper vom Kaninchen in einer Konzentration von 2,5 bis 2,7 mg/ml mit einem Titer von 5 Einheiten/ml enthält.

5. Verfahren zur Bestimmung der Konzentration von Apo B in einer Probe von Blut, Plasma oder Serum, wobei man die Probe mit einer Dispersion von Partikeln, die an Antikörper gegen Apolipoprotein B gebunden sind, mischt, die Zeit bis zur Agglutination bestimmt und die zugehörige Konzentration von Apo B einer Vergleichskurve entnimmt dadurch gekennzeichnet, daß die Menge an Antikörper so gewählt wird, daß bei einer Konzentration von Apo B, die oberhalb des klinischen Normalbereichs liegt, die Agglutination zu einem späteren Zeitpunkt eintritt als die Agglutination bei einer Konzentration im klinischen Normalbereich.

6. Verfahren nach Anspruch 5, dadurch gekennzeichnet, daß die Menge Antikörper, die an die Partikel gebunden ist und das Mischungsverhältnis von Dispersion und Probe so gewählt sind, daß die Mischung nach etwa zwei Minuten agglutiniert, wenn die Probe 170 mg Apo B in 100 ml enthält, und daß sie nicht agglutiniert, wenn die Probe mehr Apo B enthält.

**Patentansprüche für folgenden Vertragsstaat : ES**

1. Verfahren zur Bestimmung der Konzentration von Apolipoprotein B (Apo B) in einer Probe von Blut, Plasma oder Serum, wobei man die Probe mit einer Dispersion von Partikeln, an die Antikörper gegen Apo B gebunden sind, mischt, die Zeit bis zur Agglutination bestimmt und die zugehörige Konzentration von Apo B einer Vergleichskurve entnimmt dadurch gekennzeichnet, daß die Menge an Antikörper so gewählt wird, daß bei einer Konzentration von Apo B, die oberhalb des klinischen Normalbereichs liegt, die Agglutination zu einem späteren Zeitpunkt eintritt als die Agglutination bei einer Konzentration im klinischen Normalbereich.

2. Verfahren nach Anspruch 1, dadurch gekennzeichnet, daß die Menge Antikörper, die an die Partikel gebunden ist, und das Mischungsverhältnis von Dispersion und Probe so gewählt sind, daß die Mischung nach etwa zwei Minuten agglutiniert, wenn die Probe 170 mg Apo B in 100 ml enthält, und daß sie nicht agglutiniert, wenn die Probe mehr Apo B enthält.

**Claims**
**Claims for the following Contracting States : AT, BE, CH, DE, FR, GB, IT, LI, LU, NL, SE**

1. An agent for the determination of apolipoprotein B (Apo B) in blood, plasma or serum by an agglutination method, containing a dispersion of particles to which antibodies against Apo B are bound, and, where appropriate, stabilizing additives, wherein the amount of antibody is selected such that, where the concentration of Apo B is above the normal clinical range, agglutination occurs at a later time than the agglutination where the concentration is in the normal clinical range.

2. The agent as claimed in claim 1, which additionally contains a protein or a detergent.

3. The agent as claimed in claim 1, which contains 5 to 15 mg/ml of dispersed polystyrene latex having a particle size of from 200 to 800 nm and antibodies in a concentration of 2.2 to 3.3 mg/ml with a titre unit of 4 to 6.

4. The agent as claimed in claim 1, which contains 8 to 10 mg/ml of polystyrene latex having a particle size of 500 to 700 nm as well as rabbit IgG antibodies in a concentration of 2.5 to 2.7 mg/ml with a titre of 5 units/ml.

5. A method for the determination of the concentration of Apo B in a sample of blood, plasma or serum, where the sample is mixed with a dispersion of particles to which antibodies against apolipoprotein B are bound, the time until agglutination takes place is determined and the relevant concentration of Apo B is taken from a reference curve, wherein the amount of antibody is selected such that, where the concentration of Apo B is above the normal clinical range, agglutination occurs at a later time than the agglutination where the concentration is in the normal clinical range.

**6.** The method as claimed in claim 5, wherein the amount of antibody which is bound to the particles, and the mixing ratio of dispersion and sample, are selected such that the mixture agglutinates after about two minutes when the sample contains 170 mg of Apo B in 100 ml, and such that it does not agglutinate when the sample contains more Apo B.

**Claims for the following Contracting State : ES**

**1.** A method for the determination of the concentration of apolipoprotein B (Apo B) in a sample of blood, plasma or serum, where the sample is mixed with a dispersion of particles to which antibodies against apolipoprotein B are bound, the time until agglutination takes place is determined and the relevant concentration of Apo B is taken from a reference curve, wherein the amount of antibody is selected such that, where the concentration of Apo B is above the normal clinical range, agglutination occurs at a later time than the agglutination where the concentration is in the normal clinical range.

**2.** The method as claimed in claim 1, wherein the amount of antibody which is bound to the particles, and the mixing ratio of dispersion and sample, are selected such that the mixture agglutinates after about two minutes when the sample contains 170 mg of Apo B in 100 ml, and such that it does not agglutinate when the sample contains more Apo B.

**Revendications**
**Revendications pour les Etats contractants suivants : AT, BE, CH, DE, FR, GB, IT, LI, LU, NL, SE**

**1.** Agent pour la détermination de l'apolipoprotéine B (Apo B) dans le sang, le plasma ou le sérum, au moyen d'un procédé d'agglutination, contenant une dispersion de particules auxquelles sont liés des anticorps dirigés contre l'Apo B, et éventuellement des additifs stabilisants, caractérisé en ce que l'on choisit la quantité d'anticorps de manière que dans le cas d'une concentration d'Apo B qui est supérieure à la plage normale clinique, l'agglutination apparaisse à un moment plus tardif que l'agglutination dans le cas d'une concentration dans la plage normale clinique.

**2.** Agent selon la revendication 1, caractérisé en ce qu'il contient en outre une protéine ou un détergent.

**3.** Agent selon la revendication 1, caractérisé en ce qu'il contient de 5 à 15 mg/ml d'un latex de polystyrène dispersé ayant une taille de particules de 200 à 800 nm et des anticorps à une concentration de 2,2 à 3,3 mg/ml, ayant un titre de 4 à 6 unités.

**4.** Agent selon la revendication 1, caractérisé en ce qu'il contient de 8 à 10 mg/ml d'un latex de polystyrène ayant une taille de particules de 500 à 700 nm, ainsi que des anticorps IgG de lapin à une concentration de 2,5 à 2,7 mg/ml, ayant un titre de 5 unités/ml.

**5.** Procédé pour la détermination de la concentration d'Apo B dans un échantillon de sang, plasma ou sérum, dans lequel on mélange l'échantillon avec une dispersion de particules qui sont liées à des anticorps dirigés contre l'apolipoprotéine B, on détermine le temps jusqu'à l'agglutination et on déduit d'une courbe de référence la concentration correspondante d'Apo B, caractérisé en ce que l'on choisit la quantité d'anticorps de manière que dans le cas d'une concentration d'Apo B qui est supérieure à la plage normale clinique, l'agglutination apparaisse à un moment plus tardif que l'agglutination dans le cas d'une concentration dans la plage normale clinique.

**6.** Procédé selon la revendication 5, caractérisé en ce que l'on choisit la quantité d'anticorps qui est liée aux particules et le rapport de mélange de la dispersion et de l'échantillon, de manière qu'au bout d'environ 2 minutes le mélange s'agglutine lorsque l'échantillon contient 170 mg d'Apo B dans 100 ml, et qu'il ne s'agglutine pas lorsque l'échantillon contient une plus grande quantité d'Apo B.

**Revendications pour l'Etat contractant suivant : ES**

**1.** Procédé pour la détermination de la concentration de l'apolipoprotéine B (Apo B) dans un échantillon de sang, de plasma ou de sérum, dans lequel on mélange l'échantillon avec une dispersion de particules qui sont liées à des anticorps dirigés contre l'Apo B, on détermine le temps jusqu'à l'agglutination et on déduit d'une courbe de référence la concentration correspondante d'Apo B, caractérisé en ce que l'on choisit la quantité d'anticorps de manière que dans le cas d'une concentration de l'Apo B qui est supérieure à la plage normale clinique, l'agglutination apparaisse à un moment plus tardif que l'agglutination dans le cas d'une concentration dans la plage normale clinique.

2. Procédé selon la revendication 1, caractéirsé en ce que l'on choisit la quantité d'anticorps qui est liée aux particules et le rapport de mélange de la dispersion et de l'échantillon, de manière qu'au bout d'environ 2 minutes le mélange s'agglutine lorsque l'échantillon contient 170 mg d'Apo B dans 100 ml, et qu'il ne s'agglutine pas lorsque l'échantillon contient une plus grande quantité d'Apo B.

APO-B in mg/dl

Agglutinationszeitpunkt (sec)

EP 0 311 094 B1